# EUROPEAN PATENT SPECIFICATION

(11) **EP 3 485 045 B1**
(45) Date of publication and mention of the grant of the patent: **12.08.2020**
(21) Application number: 17742954.5
(22) Date of filing: 04.07.2017
(51) Int. Cl.: C12R 1/885, A01N 63/30

(54) **SUPERIOR ANTAGONISTIC TRICHODERMA SPECIES FOR PRESERVING PLANT MATERIAL AND WOOD PRODUCTS**
ERHÖHTE ANTAGONISTISCHE TRICHODERMA SPEZIEN ZUR KONSERVIERUNG VON PFLANZEN- UND HOLZMATERIAL
ESPÈCES SUPÉRIEURS ANTAGONISTES DE TRICHODERMA CAPABLE DE PRÉSERVER DU MATÉRIEL VÉGÉTAL OU DES PRODUITS À BASE DE BOIS

(30) Priority: 13.07.2016 EP 16179168
(43) Date of publication of application: 22.05.2019
(73) Proprietor: EMPA Eidgenössische Materialprüfungs- und Forschungsanstalt, 8600 Dübendorf NH (CH)
(72) Inventor: SCHWARZE, Francis, 9052 Niederteufen (CH); RIBERA-REGAL, Javier, 9014 St. Gallen (CH)
(74) Representative: Kasche, André
(86) International application number: PCT/EP2017/066571
(87) International publication number: WO 2018/011007

(56) References cited:
- EP-A1- 0 387 640
- WO-A1-2011/161404
- WO-A2-2012/140304
- GB-A- 2 239 800
- US-A- 4 713 342
- US-A1- 2014 363 871

## Description

The present invention is directed to live *Trichoderma harzianum* EMPA 720 or live *Trichoderma koningiopsis* EMPA 723, a composition of these further comprising at least one physiologically acceptable carrier compound, and a method for treating soil and/or plant material.

### Background

*Trichoderma* is a genus of fungi that is present in all soils, where they are the most prevalent culturable fungi. Many species in this genus can be characterized as opportunistic avirulent plant symbionts. Several strains of *Trichoderma* have been developed as biocontrol agents against fungal diseases of plants. The various mechanisms include antibiosis, parasitism, inducing host-plant resistance, and competition. Most biocontrol agents are from the species *T*. *harzianum*, *T*. *viride* and *T*. *hamatum*.

Copper is a commonly used cheap and effective heavy metal fungicide for the prevention and treatment of fungal rot in wood products, in particular wood products that have direct soil contact such a wooden poles and wooden groundings. Over decades copper-tolerant fungi have emerged which also produce oxalic acid, a potent copper chelator that will increase copper leaching from the wood product. Toxic chromium is frequently added to copper fungicides in order to counteract copper leaching by binding copper to wood fibers.

JP2012/095570 A teaches live *Trichoderma* immobilized on alkali charcoal in the ground to delay fungal wood decay.

GB2239800 A discloses the use of specific *Trichoderma* species, in particular *T*. *harzianum,* for treating or preventing *Serpula lacrymans* infections in wood (dry rot).

The objective underlying the present invention is the provision of improved means and methods for plant and wood product preservation.

In a first aspect, the above objective is solved by live *Trichoderma* selected from the group consisting of (i) *Trichoderma harzianum* EMPA 720 as deposited under accession number CBS140589 and (ii) *Trichoderma koningiopsis* EMPA 723 as deposited under accession number CBS 141192.

It was surprisingly found that *Trichoderma harzianum* EMPA 720 and *Trichoderma koningiopsis* EMPA 723 bear an exceptionally high lethal effect of 100% on seemingly all fungi responsible for wood rot, in particular on copper-tolerant fungi (see Example 1 below). Other fungi and other *Trichoderma* spp. do not show this broad spectrum of highly lethal effects on fungi and/or copper-tolerant fungi responsible for wood rot. Therefore, the *Trichoderma* of the present invention represent improved means for plant and wood product preservation.

*Trichoderma harzianum* EMPA 720 was deposited under accession number CBS140589 and *Trichoderma koningiopsis* EMPA 723 was deposited under accession number CBS 141192 at the *Centraalbureau voor Schimmelcultures* (CBS) in the Netherlands by MycoSolutions GmbH, Untere Böhlstrasse 7, 9052 Niederteufen, Switzerland, on the same terms as those laid down in the Budapest Treaty on the International Recognition of the Deposit of Microorganisms for the purpose of Patent Procedure. MycoSolutions GmbH has given its unreserved and irrevocable consent to the deposited material being made available to the public as evidenced by the Statement of Authorization and Consent filed with the European Patent Office.

According to the present invention, the terms "live *Trichoderma harzianum* EMPA 720" and *"Trichoderma koningiopsis* EMPA 723" encompass all components of *Trichoderma* EMPA 720/723 that are or may lead to living and reproducing *Trichoderma* 720/732 fungi, in particular mycelium, spores, conidia, conidiophores, phialides, synanamorphs, chlamydospores and hyphae.

In a preferred embodiment, the present invention is directed to spores of the live *Trichoderma harzianum* EMPA 720 or live *Trichoderma koningiopsis* EMPA 723.

In a second aspect, the present invention relates to a composition comprising (i) live *Trichoderma harzianum* EMPA 720 as deposited under accession number CBS140589 or live *Trichoderma koningiopsis* EMPA 723 as deposited under accession number CBS 141192, and (ii) at least one physiologically acceptable carrier compound.

According to the present invention, a physiologically acceptable carrier compound for use in the present invention is any non-toxic compound/composition for carrying the inventive *Trichoderma,* e.g. for adding volume, improving handling and administration to soil or wood products, etc. which carrier compound preferably supports viability or even improves growth of the inventive fungi and preferably also supports but does not prevent the fungicidal action on fungi responsible for wood rot. Furthermore, the carrier compound is preferably bio-compatible, i.e. does preferably not have toxic effects on the environment and is preferably made from organic materials. Additionally, the acceptable carrier compound of the present invention can preferably support, more preferably synergistically support the fungicidal activity and/or plant and wood product-preserving activity of the inventive *Trichoderma*.

In a preferred embodiment, the composition of the present invention is a composition, wherein the at least one physiologically acceptable carrier compound is selected from the group consisting of (i) char, preferably char selected from the group consisting of biochar, activated biochar, char of deciduous trees, preferably trees selected from the group consisting of beech trees, ash trees, maple trees, oak trees, sallow trees, whitethorn trees, hazelnut trees, and alder trees; more preferably char prepared at more than 200 °C, preferably more than 300 °C, more preferably more than 400 °C, most preferably between 400 and 700 °C; (ii) maize granules or corn granules, (iii) compost, preferably vegetable compost, more preferably leaves compost, (iv) flower soil, (v) talcum powder, (vi) vermiculite, (vii) wood dust or wood chips, preferably wood dust or wood chips of hardwoods, more preferably wood dust or wood chips from deciduous trees, preferably from trees selected from the group consisting of *Picea abies, Abies alba, Fagus sylvatica, Fraxinus excelsior* and *Acer pseudoplatanus;* and (viii) mixtures of any of (i) to (vii).

Char or biochar for use in the present invention is preferably characterized in that it improves the chemical-physical soil features, for example water holding capacity, nutrient availability and results in long-term improvement of soil fertility. Also, the carbonization process can cause a long-term cleavage of harmful CO₂. Similar to clay, biochar preferably has a huge surface area, to which a large amount of polar (water) or charged molecules can be adsorbed. Due to its high porosity, biochar can preferably absorb five times the amount of its own weight of water and dissolved nutrients can be taken up. This property is called the adsorption capacity (AK) of biochar, which depends on the one hand on the pyrolyzed biomass and on the other hand on the pyrolysis temperature. Biochar can be advantageously used in dry locations (for example on urban sites) with weakly to moderately heavy soils. For these locations, biochar can act as a water and nutrient buffer and in the long run improve soil fertility and increase humus formation. In addition to its use as nutrient supplier, biochar can be used as a habitat for microorganisms (see Examples 1 and 2). Because of the above properties, biochar in combination with the inventive fungi can effectively and synergistically reduce the inoculum of wood-destroying fungi, preferably of copper tolerant fungi in the soil and indirectly prolong the life of trees, and service life of wood, preferably of wood that has been impregnated with copper-based wood preservatives.

In a further preferred embodiment, the composition of the present invention comprises at least 10⁴, preferably at least 10⁵, most preferably 10⁶-10⁸ spores of live *Trichoderma harzianum* EMPA 720 or live *Trichoderma koningiopsis* EMPA 723 per gram of the at least one carrier compound.

In a further preferred embodiment, the composition of the present invention optionally further comprises a compound selected from the group consisting of a binder, preferably gelatine or alginate; sugar, preferably glucose; water; a nitrogen source, preferably urea, more preferably amounting to about 3.5% by weight nitrogen in the total composition; a phosphorous source, preferably P₂O₅, more preferably amounting to about 1% by weight P₂O₅ in the total composition; a potassium source, preferably K₂O, more preferably amounting to about 1% by weight K₂O in the total composition; fungal nutrients, preferably mineral supplements, more preferably mineral supplements at concentrations of 1% by weight or less, most preferably Zn²⁺, Mn²⁺, CU²⁺, and Fe²⁺; and a pH-stabilizer, preferably H₃PO₄ and/or NaHCO₃.

In addition to the carrier compound, the composition of the present invention may optionally be supplemented with compounds that improve the physical properties of the composition or that facilitate the handling of the composition. For example, binders can reduce an inventive composition's tendency to raise dust, which would be undesired during manufacturing, packaging or application. Also, the inventive composition may optionally comprise nutrients for the fungi of the present invention to assist their growth and/or proliferation, for example nitrogen, phosphorous and/or potassium sources. Furthermore, the inventive composition optionally comprises further fungal growth factors such as sugars or salts. Additionally, the inventive composition can optionally comprise agents that control, modify or stabilize the pH value of the composition, preferably to ensure that the inventive fungi are granted viable conditions for growth and/or proliferation.

In a further preferred embodiment, the composition of the present invention comprises (i) live *Trichoderma harzianum* EMPA 720 or live *Trichoderma koningiopsis* EMPA 723; (ii) maize granules; and (iii) biochar, preferably biochar of woods selected from the group consisting of beech trees, maple trees and ash trees, preferably prepared at temperatures between 450 °C and 500 °C, wherein about one gram of (ii) is inoculated with 10⁴-10⁸ spores of live *Trichoderma harzianum* EMPA 720 or live *Trichoderma koningiopsis* EMPA 723, and wherein the weight ratio of components (ii) to (iii) is preferably at least about 1:10, more preferably about 5:10, most preferably from 1:10 to 1:1.

In a third aspect, the present invention is directed to a use of live *Trichoderma harzianum* EMPA 720 or live *Trichoderma koningiopsis* EMPA 723 or a composition according to the present invention for treating soil and/or plant material against (a) plant and/or wood rot, and/or (b) infestation of copper-resistant fungi in soil and/or plant material.

Preferred dosages of the inventive composition are about 1 g of carrier compound per litre or m² of surface to be treated against wood-rot, and/or about 1 kg of carrier compound per m³ of volume to be treated against wood-rot. However, the skilled person is well aware that the dosage of the composition will also depend on the gravity of the fungi infection, the susceptibility of the treated soil and/or plant material to the infectious fungi, whether or not the soil and/ or plant material was pre-treated with other protective agents and local environmental conditions, for example precipitation, dryness, wind, etc.

In a preferred embodiment, the use according to the present invention is directed to plant material selected from the group consisting of crop plants, preferably tomatoes, cucumbers, eggplants, strawberries, cacao plants, oil palms, vines, wheat, tea plants and coffee plants; wood and wood products, preferably selected from the group consisting of trees, timber, wood poles, wood palisades and wood groundwork.

The composition for use according to the present invention is especially beneficial for use in organic farming. Because the use of the present composition preferably does not involve any synthetic chemicals or potentially harmful agents such as synthetic fungicides or heavy metals such as copper or chromium, plants can be protected organically against undesired fungi using natural products only.

In a further preferred embodiment, the use according to the present invention is directed to copper and/or chromium-treated plant material, preferably a wood pole or wood groundwork.

It has been surprisingly found that the inventive composition is exceptionally effective in protecting plant material against fungal infections that was pre-treated with copper and/or chromium-comprising agents and is therefore surprisingly effective against copper-tolerant fungi (see Example 3 below).

In many cases, fungi have become copper-tolerant and preserving with copper-comprising agents is not effective. Furthermore, copper may leak into the environment not only due to natural diffusion, but also because copper-tolerant fungi express copper-chelating oxalic acid at elevated amounts, which acid increases the leaching of copper from the preserved material into the environment. The leached copper concentrations were always higher for chromium-free wood preservatives (for example Wolmanit®CX-8 (BASF, Germany) and lmpralit®-KDS (Rütgers Organics, Germany) than for the chromium-containing wood preservatives (for example CC = chromium and copper-containing preservatives and CCB = chromium, copper and boron-containing preservatives) because chromium binds copper chemically to the wood fiber. However, chromium is even more toxic than copper if it leaches into the environment. Therefore, the use of chromium-containing preservatives is not desirable from an environmental point of view and may be restricted or prohibited. First manufacturers of chromium-based wood preservatives for wood impregnation have started not to use chromium in their formulations. But the increased use of chromium-free wood preservatives will also result in an increase of copper-leaching from wood exposed to the environment.

It was surprisingly found that the inventive *Trichoderma* have a negative effect on the oxalic acid production of copper-tolerant fungi (see Example 4 below) and can thus reduce the amount of copper that leaches from the preserved material. Furthermore, the inventive composition surprisingly showed that biochar further increases the anti-leaching effect of the composition by safely absorbing copper before it further diffuses into the environment (see Example 5 below).

Therefore, it is also preferred that the inventive composition is used together with or in addition to copper-containing preservatives in order to (i) further increase the anti-fungal efficacy of the copper-containing preservative, (ii) treat (undetected) copper tolerant fungi and to (iii) prevent the leaching of the copper from the preserved material into the environment.

In a fourth aspect, the present invention is directed to a method for treating soil and/or plant material against (a) plant and/or wood rot, and/or (b) infestation of copper-resistant fungi in soil and/or plant material, comprising the steps of (i) providing live *Trichoderma harzianum* EMPA 720 or live *Trichoderma koningiopsis* EMPA 723, spores and/or a composition according to the present invention; and (ii) applying the fungus, spores and/or composition of (i) to soil and/or plant material.

In a preferred embodiment, the fungus, spores and/or composition of the inventive method are applied to soil in the vicinity of plant material of interest, preferably at a distance of within 1 m, more preferably of at most 2, 5, 10 or 15 m, most preferably of at most 20 m.

In a further preferred embodiment, the plant material for use in the inventive method is selected from the group consisting of crop plants, preferably tomatoes, cucumbers, eggplants, strawberries, cacao plants, oil palms, vines, wheat, tea plants and coffee plants; wood and wood products, preferably selected from the group consisting of trees, timber, wood poles, wood palisades and wood groundwork.

In another preferred embodiment, the plant material for use in the inventive method is a copper and/or chromium-treated plant material, preferably a wood pole or wood groundwork.

In a further preferred embodiment, the composition of the inventive method comprises at least one physiologically acceptable carrier compound selected from the group consisting of (i) char, preferably char selected from the group consisting of biochar, activated biochar, char of deciduous trees, preferably trees selected from the group consisting of beech trees, ash trees, maple trees, oak trees, sallow trees, whitethorn trees, hazelnut trees, and alder trees; more preferably char prepared at more than 200 °C, preferably more than 300 °C, more preferably more than 400 °C, most preferably between 400 and 700 °C, (ii) maize granules or corn granules, (iii) compost, preferably vegetable compost, more preferably leaves compost; (iv) flower soil, (v) talcum powder, (vi) vermiculite, (vii) wood dust or wood chips, preferably wood dust or wood chips of hardwoods, more preferably wood dust or wood chips from deciduous trees, preferably from trees selected from the group consisting of *Picea abies, Abies alba, Fagus sylvativca, Fraxinus excelsior* and *Acer pseudoplatanus;* and (viii) mixtures of any of (i) to (vii).

In a further preferred embodiment, the method of the present invention is a method for preventing and/or treating an infestation of copper-resistant fungi in soil and/or plant material.

The invention has been described generally and also with emphasis upon preferred embodiments and will be further illustrated by the following examples, none of which should be construed to limit the scope of the invention beyond the scope of the appended claims.

### Figures

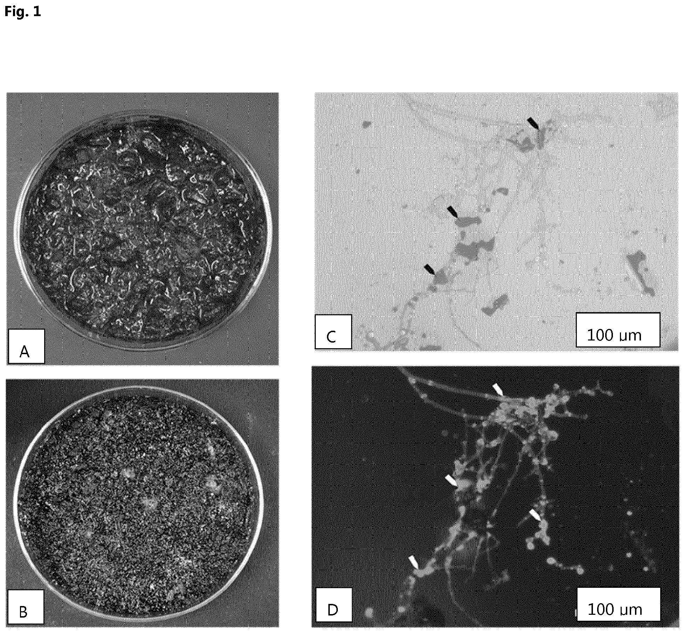

**Fig. 1** shows untreated biochar (**A**) and biochar incubated for two weeks with *T. harzianum* EMPA 720 (**B**). Fig. **1C** is a micrograph taken with a light microscope showing biochar incubated for two weeks with *T. harzianum* EMPA 720. The dark structures (arrows) consist of biochar which has been colonised by filamentous cells (hyphae) of *T. harzianum* EMPA 720. Fig. **1D** is an image of the same section shown in Fig. 1C taken with a fluorescence microscope. The spores and hyphae (white arrows) of the BFP (blue fluorescent mutant) of *T. harzianum* EMPA 720 appear bright blue (light grey on Fig. 1).

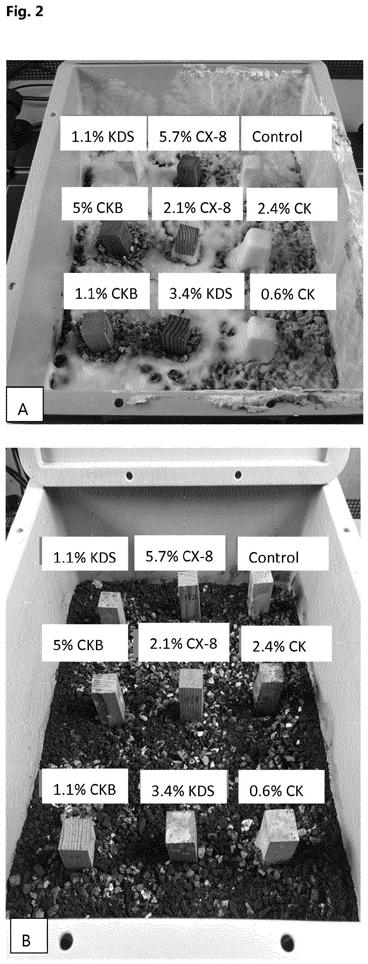

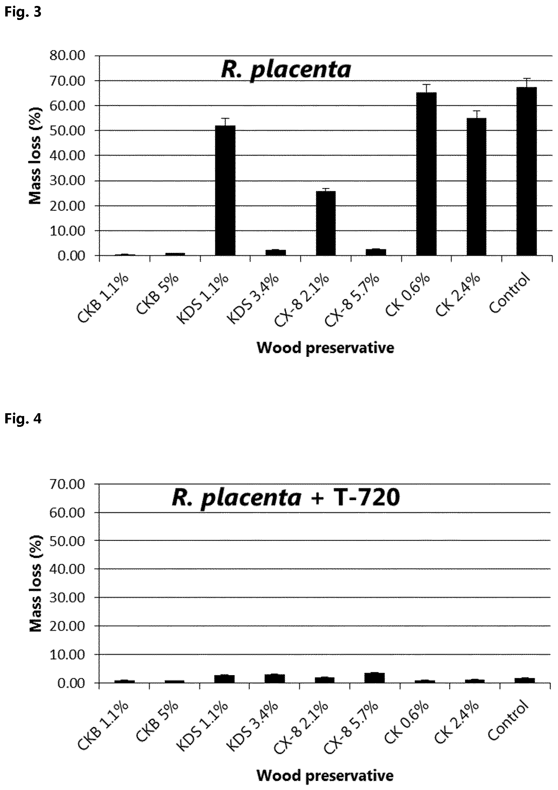

**Fig. 2A** shows boxes with impregnated mini wood stakes (wood preservative and applied concentrations are denoted for the respective stakes) after 36 weeks incubation. The following preservatives were investigated CX-8: Wolmanit®CX-8 (BASF, Germany); KDS: lmpralit®-KDS (Rütgers Organics, Germany); CK: lmpralit®-CK (Rütgers Organics, Germany); CKB: lmpralit®-CKB (Rütgers Organics, Germany).Mini wood stakes were not pre-treated with *T. harzianum* EMPA 720. Mass losses are also discernable in Fig. 3. Fig. **2B** shows boxes with impregnated mini wood stakes (wood preservative and applied concentrations are denoted for the respective stakes) after 36 weeks incubation. Mini wood stakes were pre-treated with spore suspensions of *T*. *harzianum* EMPA 720 before insertion into the box. Mass losses are also discernable in Fig. 4.
**Fig. 3** depicts mass losses by the copper-tolerant fungus *Rhodonia placenta* in impregnated mini wood stakes after 9 months incubation without pre-treatment with *T. harzianum* EMPA 720.
**Fig. 4** depicts mass losses by the copper-tolerant fungus *Rhodonia placenta* in impregnated mini wood stakes after 9 months incubation and a pre-treatment with spores suspensions of *T*. *harzianum* EMPA 720.

### Examples

### Example 1: Dual culture tests - Antifungal properties of Trichoderma spp.

In order to determine *in vitro* suppression of *Trichoderma* species against the wood-destroying fungi, dual culture tests with different combination (n = 6) of *Trichoderma* species and copper tolerant fungi (Table 1 below) were performed. The agar disc method was carried out on a common growth media 2% MEA. Mycelial discs (5 mm) were removed from 1-week-old MEA cultures of each of the five wood-decay fungi and placed equidistantly at the margin of Petri dishes (90 mm) containing 2% MEA and 30% Biochar (Carbon Gold, UK). These were incubated at 25(±1) °C and 70% relative humidity for 3-4 days. Next, discs (5 mm) were removed from the margins of actively growing 1-week-old cultures of the *Trichoderma* species and placed at opposite sides of the dish, and incubated in the dark at 25(±1) °C and 70% relative humidity for 4 weeks. Petri dishes without *Trichoderma* were used as the control. The ability of *Trichoderma* species to eliminate the wood decay fungi (lethal effect) during the four week incubation period was evaluated by aseptically transferring 5-mm discs from test plates to MEA with 2 mL thiabendazole (2-(40-thiazolyl)- benz-imidazole; Merck, Darmstadt, Germany; 0.46 mg dissolved in lactic acid). Thiabendazole-MEA suppresses the growth of *Trichoderma* species but allows growth of wood-decay fungi (Sieber T. (1995) Pyrenochaeta ligni-putridi sp. nov., a new coelomycete associated with butt rot of Picea abies in Switzerland. Mycological Research 99(3):274-276).

The lethal effect of *Trichoderma* species was expressed as a percentage of the wood-decay fungi which were eliminated (Table 1 below). The studies showed that *Trichoderma harzianum* EMPA 720 showed a 100% lethal effect against all copper tolerant fungi tested.**Table 1**. Classification of the lethal effect of different *Trichoderma* species against different copper tolerant fungi on malt extract amended with 30 % biochar.

| **Cu-tolerant fungi** | **% Lethal effec**t | | | | |
|---|---|---|---|---|---|
| | *T. atroviride* (685) | *T*. *harzianum* (720) | *T. harzianum* (721) | *T. atroviride* (722) | *T*. *koningiopsis* (723) |
| *G*. *sepiarium* (592) | 100 | 100 | 80 | 60 | 80 |
| *S. brinkmannii* (417) | 20 | 100 | 40 | 40 | 20 |
| *A. serialis* (364) | 85 | 100 | 100 | 95 | 80 |
| *A. seriatis* (481) | 100 | 100 | 85 | 80 | 100 |
| *A. serialis* (716) | 100 | 100 | 100 | 100 | 100 |
| *S. himantioides* (731) | 65 | 100 | 55 | 60 | 65 |
| *A. serialis* (165) | 100 | 100 | 100 | 100 | 100 |
| *S. himantioides* (581) | 80 | 100 | 80 | 40 | 20 |
| *A. serialis* (049) | 100 | 100 | 85 | 80 | 100 |
| *S. himantioides* (643) | 70 | 100 | 55 | 65 | 80 |
| *F. vaillantii* (332) | 90 | 100 | 85 | 70 | 85 |
| *F. vaillantii* (774) | 80 | 100 | 60 | 65 | 75 |
| *A. serialis* (150) | 100 | 100 | 100 | 100 | 100 |
| *N. lepideus* (428) | 100 | 100 | 100 | 100 | 100 |

### Example 2: Growth and sporulation rates of Trichoderma spp. on biochar

For the determination of fungal growth, biochar was inoculated with 10⁸ / ml spores of *Trichoderma harzianum* EMPA 720 (n = 3). To determine the influence of different substrates on the number of spores produced, two biochar substrates were used. (1) Maize powder and biochar (ratio 1:10) (Merck (Germany), Carbon Gold, UK) and (2) pure biochar (Carbon Gold, UK) without a carbon source. After two weeks incubation at 25 °C and a relative humidity of 65% the substrates were mixed and 1 gram biochar was dissolved in 1 liter of H₂O and the number of spores of *T. harzianum* EMPA 720 were measured in a Neubauer chamber. The studies showed that in the mixture of biochar and maize powder 492,000 spores / ml and in the substrate of pure biochar 217,500 spores / ml could be detected. To determine the growth of *T*. *harzianum* EMPA 720 in the biochar, a BFP (bluish fluorescent protein) mutant of the fungus was used. For this purpose, pCAMB (CAMBIA, Canberra, Australia) binary vector containing a hygromycin resistance gene and the GFP (Green fluorescent protein) gene from pCT74 (Lorang, J.M., Tuori, R.P., Martinez, J.P., Sawyer. T.L., Redman, R.S., Rollins, J.A., Wolpert, T.J., Johnson, K.B., Rodriguez, R.J., Dickman, M.B., Ciuefftti, L.M. (2001) Green fluorescent protein is lighting up fungal biology. Appl. Environ. Microbiol. 67, 1987-1994.) was constructed. The resulting vector (pCAMBgfp) was introduced into *Agrobacterium tumefaciens* strain AGL-1 and transformed into *Trichoderma-GFP* as described by De Groot, M.J.A., Bundock, P., Hooykaas, P.J.J., Beijersbergen, A.G.M. (1998) Agrobacterium tumefaciens-mediated transformation of filamentous fungi. Nature Biotechnology. 16:839-842.

Detection of hyphal growth and spore formation by of *T. harzianum* EMPA 720 in the biochar was performed using samples taken from the inoculated biochar under the fluorescence microscope. Untreated biochar served as a control (Fig. 1A). The studies showed that after just two weeks, the biochar was colonized by the filamentous cells of *T. harzianum* EMPA 720 and large quantities of spores were formed (Fig. 1B).

### Example 3: Determination of fungicidal efficacy of wood preservatives after 36 weeks of exposure to copper-tolerant fungi according to ENV 807

The performance of the artificial interaction tests was carried out in a modified form according to the European Standard EN 113 (1996). For this purpose mini-wood stakes with dimensions of 8 x 2 x 2 cm were excised from *Picea abies* sapwood and treated with different wood preservatives (see Table 2 below).

**Table 2. List of used wood preservatives**

| **Wood preservatives** | **Abreviation** |
|---|---|
| CC (3.8 kg/m3) | 0.6% CC |
| CC (21.3 kg/m3) | 2.4% CC |
| CCB (8.1 kg/m3) | 1.1% CCB |
| CCB (40.3 kg/m3) | 5.0% CCB |
| KDS (8.3 kg/m3) | 1.1% KDS |
| KDS (26.1kg/m3) | 3.4% KDS |
| CX-8 (15.1 kg/m3) | 2.1% CX-8 |
| CX-8 (43.3 kg/m3) | 5.8% CX-8 |

The abbreviations in Table 2 and in the present description correspond to the following chemicals. CC: chromium and copper-containing preservative; CCB: chromium, cooper and boron-containing preservative; CX-8: Wolmanit®CX-8 (BASF, Germany); KDS: lmpralit®-KDS (Rütgers Organics, Germany).

For assessing the preventive effect of *T. harzianum* EMPA 720 in wood, that has been colonized by copper-tolerant a special substrate for long-term experiments was produced. The substrate used for inoculation of the wood stakes with copper-tolerant fungi consisted of 30% biochar, 68% vermiculite and 2% wood dust from *Picea abies* wood (Carbon Gold, UK) and 50% w/v distilled H₂O. The substrate was sterilized for 20 min at a temperature of 120 °C in an autoclave and then inoculated with sawdust that had been incubated with the copper tolerant fungus *R. placenta* at 28 °C/95% relative humidity for four weeks. After incubation for three months the mini wood stakes were placed for 36 weeks at 28 °C and 95% relative humidity into boxes with the substrate enriched with the copper-tolerant fungus (Fig. 2A). In a second box impregnated mini wood stakes were dipped in aqueous spore suspensions of 10⁷ CFU/ml and then placed into the substrate enriched with copper-tolerant fungi for 36 weeks (Fig. 2B). Based on the determination of the mass loss, the preventive effect of *T. harzianum* EMPA 720 against copper-tolerant fungi was determined.

Fig. 2A shows that after 36 weeks recorded mass losses in the CCB impregnated mini wood stakes were the lowest (0.58% and 1.02%). The highest mass loss was recorded in the untreated control samples. The CC impregnated mini wood stakes also showed very high mass losses (55.06% and 65.24%). At high concentrations, the chromium-free wood preservatives (CX-8 and KDS) showed some protection. At low concentrations, the mass loss was between 25.71-52.15% (Fig. 3). The results with the copper tolerant fungus *Rhodonia placenta* confirmed numerous field observations that the potentially toxic CCB wood preservatives that have been used in the past are to some extent efficient against copper-tolerant fungi. The CC wood preservatives are highly susceptible against copper-tolerant fungi without the co-biocide boron. The positive effect of *T. harzianum* EMPA 720 against the copper tolerant fungus *R. placenta* was very clear and impressive (Fig. 4). Regardless of the wood preservative applied and their concentrations the recorded weight losses from the impregnated mini wood were always significantly lower after treatment with *T. harzianum* EMPA 720 (Fig. 2B). On average, the recorded weight losses of impregnated mini wooden stakes were in the range of 0.82% -3.51%, respectively. Even the untreated control showed a minor mass loss of 1.71%. Thus, the preventive action of *T*. *harzianum* EMPA 720 against the copper tolerant fungus *R. placenta* was very impressive.

### Example 4: Reduction of the oxalic acid production of copper tolerant fungi by Trichoderma harzianum 720

Several authors describe that the production of oxalic acid and the presence of the wood copper oxalate crystals are an important feature of copper tolerant fungi during the detoxification of copper (Murphy RJ, Levy JF 1983. Production of copper oxalate by some copper tolerant fungi. Transactions of the British Mycological Society 81: 165-168; Sutter HP, Jones EBG, Walchli O.1984. Occurrence of crystalline hyphal sheath in Poria placenta (Fr.) Cke. Journal of Institute of Wood Science 10: 19-23; Green F, Clausen CA 2003. Copper tolerance of brown-rot fungi: time course of oxalic acid production International Biodeterioration & Biodegradation 51: 145-149; Woodward, B. M., and R. C. De Groot. 1999. Tolerance of Wolfiporia cocos isolates to copper in agar media. For. Prod. J. 49:87-94.)

For the investigation of the influence of *Trichoderma* species on the oxalic acid production of copper tolerant fungi, five different copper-tolerant fungi (*Antrodia serialis, Fibroporia vaillantii, Gloeophyllum sepiarium, Rhodonia placenta* and *Serpula himantioides*) and *Trichoderma harzianum* EMPA 720 were cultivated in 250 ml Erlenmeyer flask containing 120 ml of a liquid media with 1% malt extract for 20 days and shaken at 25 °C and 65% relative humidity at 150 rpm. The suspension was then filtered and centrifuged at 12,000 rpm for 1 min. The supernatant obtained was used to measure the oxalic acid production of the copper-tolerant fungi by means of a colorimetric assay kit (Sigma Aldrich, Switzerland). The same experiment as described above was repeated, but after 20 days, the liquid media with the copper-tolerant fungi was inoculated with 0.1 ml of *T. harzianum* EMPA 720. The Erlenmeyer flasks were incubated as described above for a further 20 days and then measured oxalic acid production (Table 2 below). The studies showed that the oxalic acid production (Table 2 below) of all copper-tolerant fungi was significantly reduced in the liquid medium by the addition of *T. harzianum* EMPA 720.

**Table 3. Oxalic acid production (in mg /l) in a fluid media after 8 weeks incubation (n=3).**

| Fungal spp. | control (mg/l) | Dual culture (mg/l) |
|---|---|---|
| *A. serialis* | 11.41 ± 1.75 | 7.60 ± 1.09 |
| *S. himatioides* | 19.82 ± 0.53 | 8.52 ± 0.85 |
| *F. vaillantii* | 18.69 ± 1.00 | 9.40 ± 0.50 |
| *R. placenta* | 10.63 ± 0.34 | 1.15 ± 0.37 |
| *G. sepirarium* | 20.12 ± 0.26 | 11.54 ± 0.30 |
| *T. harzianum* 720 | 8.06 ± 0.20 | |

### Example 5: Leaching of copper from impregnated wood and its adsorption by biochar

Three wood samples of Scots pine (*Pinus sylvestris L.*) with dimensions of 2 x 2 x 8 cm were treated with CC, CCB, CX-8 and KDS wood preservatives (Table 3 below). After a drying and fixation process for two weeks, the samples were placed into an Erlenmeyer flask with 500 ml of distilled water and 2.5 g biochar (Carbon Gold, UK). Erlenmeyer flask with impregnated wood samples and without biochar served as controls. The Erlenmeyer flasks with the wood samples were shaken for 10 days at 25 °C and 65% relative humidity at 150 rpm. The suspension was then centrifuged at 4000 rpm for 30 min and the supernatant separated from the biochar. This was then mixed with 2% HNO₃ and the copper concentration was measured by ICP-OES (Inductively coupled plasma atomic emission spectroscopy, Perkin Elmer Optima 3000, Perkin Elmer corp., United States). In addition, the copper absorption of biochar was analyzed. For this purpose, 1g biochar (Carbon Gold, UK) was added to 3 ml HNO₃ und 1 ml H₂O₂. After leaching in a microwave, the copper content by ICP-OES was determined. Suspensions without biochar served as controls.

The studies showed that even 1 gram of biochar absorbs different amounts of copper from wood during leaching exposure. Thus the copper concentration in the supernatant was reduced in the presence of biochar for all wood preservatives.

The leached copper concentrations were always higher for the chromium-free wood preservatives (CX-8 and KDS) than for the wood preservatives with chromium (CC and CCB). Leaching losses for chromium-containing wood preservatives are generally low since chromium binds copper chemically to the wood fiber.

**Table 4. ICP measurements of copper from impregnated wood after 10 days leaching exposure.**

| **Treatment** | **Cu-leaching** | **Cu-leaching** |
|---|---|---|
| | Cu in supernatant (mg/l) Without biochar | Cu in supernatant (mg/l) With 1 gram biochar |
| Control (only biochar) | - | 0.01 ± 0.00 |
| Control (only H₂O) | - | 0.01 ± 0.00 |
| Control (untreated wood) | 0.01 ± 0.00 | 0.00 ± 0.00 |
| CC (21.3 kg/m³) | 17.6 ± 0.00 | 2.4 ± 0.00 |
| CCB (40.3 kg/m³) | 28.7 ± 0.00 | 0.8 ± 0.00 |
| CX-8 (43.3 kg/m³) | 140.0 ± 0.00 | 104 ± 0.00 |
| KDS (26.1 kg/m³) | 52.5 ± 0.00 | 47 ± 0.000 |

### Summary

The present invention is directed to live *Trichoderma harzianum* EMPA 720 or live *Trichoderma koningiopsis* EMPA 723, a composition of these further comprising at least one physiologically acceptable carrier compound, and a method for treating soil and/or plant material.

## Claims

1. Live *Trichoderma* selected from the group consisting of (i) *Trichoderma harzianum* EMPA 720 as deposited under accession number CBS140589 and (ii) *Trichoderma koningiopsis* EMPA 723 as deposited under accession number CBS 141192.

2. Spores of the live *Trichoderma* of claim 1.

3. A composition comprising
(i) live *Trichoderma harzianum* EMPA 720 as deposited under accession number CBS140589 or live *Trichoderma koningiopsis* EMPA 723 as deposited under accession number CBS 141192, and
(ii) at least one physiologically acceptable carrier compound.

4. The composition according to claim 3, wherein the at least one physiologically acceptable carrier compound is selected from the group consisting of
(i) char, preferably char selected from the group consisting of biochar, activated biochar, char of deciduous trees, preferably trees selected from the group consisting of beech trees, ash trees, maple trees, oak trees, sallow trees, whitethorn trees, hazelnut trees, and alder trees; more preferably char prepared at more than 200 °C, preferably more than 300 °C, more preferably more than 400 °C, most preferably between 400 and 700 °C;
(ii) maize granules or corn granules,
(iii) compost, preferably vegetable compost, more preferably leaves compost,
(iv) flower soil,
(v) talcum powder,
(vi) vermiculite,
(vii) wood dust or wood chips, preferably wood dust or wood chips of hardwoods, more preferably wood dust or wood chips from deciduous trees, preferably from trees selected from the group consisting of *Picea abies, Abies alba, Fagus sylvatica, Fraxinus excelsior* and *Acer pseudoplatanus;* and
(viii) mixtures of any of (i) to (vii).

5. The composition according to any of claims 3 or 4, wherein the composition comprises at least 10⁴, preferably at least 10⁵, most preferably 10⁶-10⁸ spores of live *Trichoderma harzianum* EMPA 720 or live *Trichoderma koningiopsis* EMPA 723 per gram of the at least one carrier compound.

6. The composition according to any one of claims 3 to 5, wherein the composition optionally further comprises a compound selected from the group consisting of a binder, preferably gelatine or alginate; sugar, preferably glucose; water; a nitrogen source, preferably urea, more preferably amounting to about 3.5% nitrogen in the total composition; a phosphorous source, preferably P₂O₅, more preferably amounting to about 1% P₂O₅ in the total composition; a potassium source, preferably K₂O, more preferably amounting to about 1% K₂O in the total composition; fungal nutrients, preferably mineral supplements, more preferably mineral supplements at concentrations of 1% by weight or less, most preferably Zn²⁺, Mn²⁺, Cu²⁺, and Fe²⁺; and a pH-stabilizer, preferably H₃PO₄ and/or NaHCO₃.

7. The composition according to any one of claims 3 to 6, wherein the composition comprises
(i) live *Trichoderma harzianum* EMPA 720 or live *Trichoderma koningiopsis* EMPA 723;
(ii) maize granules; and
(iii) biochar, preferably biochar of woods selected from the group consisting of beech trees, maple trees and ash trees, preferably prepared at temperatures between 450 °C and 500 °C,
wherein about one gram of (ii) is inoculated with 10⁴-10⁸ spores of live *Trichoderma harzianum* EMPA 720 or live *Trichoderma koningiopsis* EMPA 723, and wherein the weight ratio of components (ii) to (iii) is preferably at least about 1:10, more preferably about 5:10, most preferably from 1:10 to 1:1.

8. Use of live *Trichoderma harzianum* EMPA 720 or live *Trichoderma koningiopsis* EMPA 723 according to claims 1 or 2 or a composition according to any of claims 3 to 7 for treating soil and/or plant material against
(a) plant and/or wood rot, and/or
(b) infestation of copper-resistant fungi in soil and/or plant material.

9. The use according to claim 8, wherein the plant material is selected from the group consisting of crop plants, preferably tomatoes, cucumbers, eggplants, strawberries, cacao plants, oil palms, vines, wheat, tea plants and coffee plants; wood and wood products, preferably selected from the group consisting of trees, timber, wood poles, wood palisades and wood groundwork.

10. The use according to any of claims 8 or 9, wherein the plant material is a copper and/or chromium-treated plant material, preferably a wood pole or wood groundwork.

11. A method for treating soil and/or plant material against (a) plant and/or wood rot, and/or (b) infestation of copper-resistant fungi in soil and/or plant material, comprising the steps of
(i) providing live *Trichoderma harzianum* EMPA 720 or live *Trichoderma koningiopsis* EMPA 723 according to claim 1, spores according to claim 2 and/or a composition according to any of claims 3 to 7; and
(ii) applying the fungus, spores and/or composition of (i) to soil and/or plant material.

12. The method according to claim 11, wherein the fungus, spores and/or composition of (i) are applied to soil in the vicinity of plant material of interest, preferably at a distance of within 1 m, more preferably of at most 2, 5, 10 or 15 m, most preferably of at most 20 m.

13. The method according to any of claims 11 or 12, wherein the plant material is selected from the group consisting of crop plants, preferably tomatoes, cucumbers, eggplants, strawberries, cacao plants, oil palms, vines, wheat, tea plants and coffee plants; wood and wood products, preferably selected from the group consisting of trees, timber, wood poles, wood palisades and wood groundwork.

14. The method according to any of claims 11 to 13, wherein the plant material is a copper and/or chromium-treated plant material, preferably a wood pole or wood groundwork.

15. The method according to any of claims 11 to 14, wherein the composition comprises at least one physiologically acceptable carrier compound selected from the group consisting of
(i) char, preferably char selected from the group consisting of biochar, activated biochar, char of deciduous trees, preferably trees selected from the group consisting of beech trees, ash trees, maple trees, oak trees, sallow trees, whitethorn trees, hazelnut trees, and alder trees; more preferably char prepared at more than 200 °C, preferably more than 300 °C, more preferably more than 400 °C, most preferably between 400 and 700 °C;
(ii) maize granules or corn granules,
(iii) compost, preferably vegetable compost, more preferably leaves compost,
(iv) flower soil,
(v) talcum powder,
(vi) vermiculite,
(vii) wood dust or wood chips, preferably wood dust or wood chips of hardwoods, more preferably wood dust or wood chips from deciduous trees, preferably from trees selected from the group consisting of *Picea abies, Abies alba, Fagus sylvatica, Fraxinus excelsior* and *Acer pseudoplatanus*; and
(viii) mixtures of any of (i) to (vii).

16. The method according to any of claims 11 to 15 for preventing and/or treating an infestation of copper-resistant fungi in soil and/or plant material.

## Patentansprüche

1. Lebende *Trichoderma* ausgewählt aus der Gruppe bestehend aus (i) *Trichoderma harzianum* EMPA 720 wie mit der Eingangsnummer CBS140589 hinterlegt und (ii) *Trichoderma koningiopsis* EMPA 723 wie mit der Eingangsnummer CBS 141192 hinterlegt.

2. Sporen der lebenden *Trichoderma* von Anspruch 1.

3. Eine Zusammensetzung umfassend
(i) lebende *Trichoderma harzianum* EMPA 720 wie mit der Eingangsnummer CBS140589 hinterlegt oder lebende *Trichoderma koningiopsis* EMPA 723 wie mit der Eingangsnummer CBS 141192 hinterlegt, und
(ii) wenigstens eine physiologisch verträgliche Trägerverbindung.

4. Die Zusammensetzung gemäss Anspruch 3, wobei die wenigstens eine physiologisch verträgliche Trägerverbindung ausgewählt ist aus der Gruppe bestehend aus
(i) Kohle, vorzugsweise Kohle ausgewählt aus der Gruppe bestehend aus Biokohle, aktivierter Biokohle, Kohle von Laubbäumen, vorzugsweise Bäume ausgewählt aus der Gruppe bestehend aus Buchen, Eschen, Ahornbäumen, Eichen, Weidenbäumen, Weissdornbäumen, Haselnussbäumen und Erlen; mehr bevorzugt Kohle, die bei mehr als 200 °C, vorzugsweise mehr als 300 °C, mehr bevorzugt mehr als 400 °C, am meisten bevorzugt zwischen 400 und 700 °C hergestellt wurde;
(ii) Maiskörnern oder Getreidekörnern,
(iii) Kompost, vorzugsweise Pflanzenkompost, mehr bevorzugt Blätterkompost,
(iv) Blumenerde,
(v) Talkumpuder,
(vi) Vermiculit,
(vii) Holzstaub oder Holzspänen, vorzugsweise Holzstaub oder Holzspäne von Harthölzern, mehr bevorzugt Holzstaub oder Holzspäne von Laubbäumen, vorzugsweise von Bäumen ausgewählt aus der Gruppe bestehend aus *Picea abies, Abies alba, Fagus sylvatica, Fraxinus excelsior* und *Acer pseudoplatanus*; und
(viii) Mischungen von irgendeinem von (i) bis (vii).

5. Die Zusammensetzung gemäss einem der Ansprüche 3 oder 4, wobei die Zusammensetzung wenigstens 10⁴, vorzugsweise wenigstens 10⁵, am meisten bevorzugt 10⁶-10⁸ Sporen von lebenden *Trichoderma harzianum* EMPA 720 oder lebenden *Trichoderma koningiopsis* EMPA 723 pro Gramm der wenigstens einen Trägerverbindung umfasst.

6. Die Zusammensetzung gemäss einem der Ansprüche 3 bis 5, wobei die Zusammensetzung optional zusätzlich eine Verbindung umfasst, ausgewählt aus der Gruppe bestehend aus einem Bindemittel, vorzugsweise Gelatine oder Alginat; Zucker, vorzugsweise Glukose; Wasser; einer Stickstoffquelle, vorzugsweise Harnstoff, mehr bevorzugt in der Menge von ungefähr 3.5% Stickstoff in der Gesamtzusammensetzung; einer Phosphorquelle, vorzugsweise P₂O₅, mehr bevorzugt in der Menge von ungefähr 1% P₂O₅ in der Gesamtzusammensetzung; einer Kaliumquelle, vorzugsweise K₂O, mehr bevorzugt in der Menge von etwa 1% K₂O in der Gesamtzusammensetzung; Pilznährstoffen, vorzugsweise Mineralstoffzusätze, mehr bevorzugt Mineralstoffzusätze in Konzentrationen von 1 Gewichts-% oder weniger, am meisten bevorzugt Zn²⁺, Mn²⁺, Cu²⁺ und Fe²⁺; und einem pH-Stabilisator, vorzugsweise H₃PO₄ und/oder NaHCO₃.

7. Die Zusammensetzung gemäss einem der Ansprüche 3 bis 6, wobei die Zusammensetzung
(i) lebende *Trichoderma harzianum* EMPA 720 oder lebende *Trichoderma koningiopsis* EMPA 723;
(ii) Maiskörner; und
(iii) Biokohle, vorzugsweise Biokohle von Hölzern ausgewählt aus der Gruppe bestehend aus Buchen, Ahornbäumen und Eschen, vorzugsweise hergestellt bei Temperaturen zwischen 450 °C und 500 °C,
umfasst,
wobei ungefähr ein Gramm von (ii) mit 10⁴-10⁸ Sporen von lebenden *Trichoderma harzianum* EMPA 720 oder lebenden *Trichoderma koningiopsis* EMPA 723 inokuliert wird, und wobei das Gewichtsverhältnis der Komponenten (ii) zu (iii) vorzugsweise wenigstens ungefähr 1:10, mehr bevorzugt ungefähr 5:10, am meisten bevorzugt von 1:10 bis 1:1 beträgt.

8. Verwendung von lebenden *Trichoderma harzianum* EMPA 720 oder lebenden *Trichoderma koningiopsis* EMPA 723 gemäss den Ansprüchen 1 oder 2 oder eine Zusammensetzung gemäss einem der Ansprüche 3 bis 7 zur Behandlung von Erde und/oder Pflanzenmaterial gegen
(a) Pflanzen- und/oder Holzfäule, und/oder
(b) Befall von kupferresistenten Pilzen in Erde und/oder Pflanzenmaterial.

9. Die Verwendung gemäss Anspruch 8, wobei das Pflanzenmaterial ausgewählt ist aus der Gruppe bestehend aus Nutzpflanzen, vorzugsweise Tomaten, Gurken, Auberginen, Erdbeeren, Kakaopflanzen, Ölpalmen, Reben, Weizen, Teepflanzen und Kaffeepflanzen; Holz und Holzprodukten, vorzugsweise ausgewählt aus der Gruppe bestehend aus Bäumen, Holz, Holzpfählen, Holzpalisaden und Holzfundamenten.

10. Die Verwendung gemäss einem der Ansprüche 8 oder 9, wobei das Pflanzenmaterial ein kupfer- und/oder chrombehandeltes Pflanzenmaterial, vorzugsweise ein Holzpfahl oder ein Holzfundament ist.

11. Ein Verfahren zur Behandlung von Erde und/oder Pflanzenmaterial gegen (a) Pflanzen-und/oder Holzfäule, und/oder (b) Befall von kupferresistenten Pilzen in Erde und/oder Pflanzenmaterial, umfassend die Schritte
(i) Bereitstellen von lebenden *Trichoderma harzianum* EMPA 720 oder lebenden *Trichoderma koningiopsis* EMPA 723 gemäss Anspruch 1, Sporen gemäss Anspruch 2 und/oder einer Zusammensetzung gemäss einem der Ansprüche 3 bis 7; und
(ii) Anwenden des Pilzes, der Sporen und/oder der Zusammensetzung von (i) auf Erde und/oder Pflanzenmaterial.

12. Das Verfahren gemäss Anspruch 11, wobei der Pilz, die Sporen und/oder die Zusammensetzung von (i) auf Erde in der Nähe von Pflanzenmaterial von Interesse, vorzugsweise in einem Abstand innerhalb von 1 m, mehr bevorzugt von höchstens 2, 5, 10 oder 15 m, am meisten bevorzugt von höchstens 20 m, angewendet werden.

13. Das Verfahren gemäss einem der Ansprüche 11 oder 12, wobei das Pflanzenmaterial ausgewählt ist aus der Gruppe bestehend aus Nutzpflanzen, vorzugsweise Tomaten, Gurken, Auberginen, Erdbeeren, Kakaopflanzen, Ölpalmen, Reben, Weizen, Teepflanzen und Kaffeepflanzen; Holz und Holzprodukten, vorzugsweise ausgewählt aus der Gruppe bestehend aus Bäumen, Holz, Holzpfählen, Holzpalisaden und Holzfundamenten.

14. Das Verfahren gemäss einem der Ansprüche 11 bis 13, wobei das Pflanzenmaterial ein kupfer- und/oder chrombehandeltes Pflanzenmaterial, vorzugsweise ein Holzpfahl oder ein Holzfundament ist.

15. Das Verfahren gemäss einem der Ansprüche 11 bis 14, wobei die Zusammensetzung wenigstens eine physiologisch verträgliche Trägerverbindung umfasst, ausgewählt aus der Gruppe bestehend aus
(i) Kohle, vorzugsweise Kohle ausgewählt aus der Gruppe bestehend aus Biokohle, aktivierter Biokohle, Kohle von Laubbäumen, vorzugsweise Bäume ausgewählt aus der Gruppe bestehend aus Buchen, Eschen, Ahornbäumen, Eichen, Weidenbäumen, Weissdornbäumen, Haselnussbäumen und Erlen; mehr bevorzugt Kohle, die bei mehr als 200 °C, vorzugsweise mehr als 300 °C, mehr bevorzugt mehr als 400 °C, am meisten bevorzugt zwischen 400 und 700 °C hergestellt wurde;
(ii) Maiskörnern oder Getreidekörnern,
(iii) Kompost, vorzugsweise Pflanzenkompost, mehr bevorzugt Blätterkompost,
(iv) Blumenerde,
(v) Talkumpuder,
(vi) Vermiculit,
(vii) Holzstaub oder Holzspänen, vorzugsweise Holzstaub oder Holzspäne von Harthölzern, mehr bevorzugt Holzstaub oder Holzspäne von Laubbäumen, vorzugsweise von Bäumen ausgewählt aus der Gruppe bestehend aus *Picea abies, Abies alba, Fagus sylvatica, Fraxinus excelsior* und *Acer pseudoplatanus*; und
(viii) Mischungen von irgendeinem von (i) bis (vii).

16. Das Verfahren gemäss irgendeinem der Ansprüche 11 bis 15 zur Vorbeugung und/oder Behandlung eines Befalls von kupferresistenten Pilzen in Erde und/oder Pflanzenmaterial.

## Revendications

1. *Trichoderma* vivant choisi dans le groupe constitué de (i) *Trichoderma harzianum* EMPA 720 tel que déposé sous le numéro d'entrée CBS 140589 et (ii) *Trichoderma koningiopsis* EMPA 723 tel que déposé sous le numéro d'entrée CBS 141192.

2. Spores du *Trichoderma* vivant selon la revendication 1.

3. Composition comprenant:
(i) le *Trichoderma harzianum* EMPA 720 vivant tel que déposé sous le numéro d'entrée CBS 140589 ou le *Trichoderma koningiopsis* EMPA 723 vivant tel que déposé sous le numéro d'entrée CBS 141192, et
(ii) au moins un composé véhicule physiologiquement acceptable.

4. Composition selon la revendication 3, dans laquelle le au moins un composé véhicule physiologiquement acceptable est choisi dans le groupe constitué de:
(i) un charbon, de préférence un charbon choisi dans le groupe constitué de: biocharbon, biocharbon activé, charbon d'arbres caducs, de préférence des arbres choisis dans le groupe constitué de: hêtres véritables, frênes, érables, chênes, saules, aubépines, noisetiers et aulnes; plus préférablement un charbon préparé à plus de 200°C, de préférence plus de 300°C, plus préférablement plus de 400°C, le plus préférablement entre 400 et 700°C;
(ii) granulés de blé d'Inde ou granulés de maïs,
(iii) un compost, de préférence un compost végétal, plus préférablement un compost de feuilles,
(iv) un sol pour fleurs,
(v) une poudre de talc,
(vi) vermiculite,
(vii) une poussière de bois ou des copeaux de bois, de préférence une poussière de bois ou des copeaux de bois provenant de bois durs, plus préférablement une poussière de bois ou des copeaux de bois provenant d'arbres caducs, de préférence provenant d'arbres choisis dans le groupe constitué de: *Picea abies, Abies alba, Fagus sylvatica, Fraxinus excelsior* et *Acer pseudoplatanus;* et
(viii) mélanges de l'un quelconque de (i) à (vii).

5. Composition selon l'une quelconque des revendications 3 ou 4, où la composition comprend au moins 10⁴, de préférence au moins 10⁵, le plus préférablement 10⁶-10⁸ spores de *Trichoderma harzianum* EMPA 720 vivant ou de *Trichoderma koningiopsis* EMPA 723 vivant par gramme du au moins un composé véhicule.

6. Composition selon l'une quelconque des revendications 3 à 5, où la composition comprend en outre optionnellement un composé choisi dans le groupe constitué de: un liant, de préférence, gélatine ou alginate; un sucre, de préférence glucose; eau; une source d'azote, de préférence urée, plus préférablement s'élevant à environ 3,5% d'azote dans la composition totale; une source de phosphore, de préférence P₂O₅, plus préférablement s'élevant à environ 1% de P₂O₅ dans la composition totale; une source de potassium, de préférence K₂O, plus préférablement s'élevant à environ 1% de K₂O dans la composition totale; des nutriments fongiques, de préférence des compléments minéraux, plus préférablement des compléments minéraux à des concentrations de 1% en poids ou moins, le plus préférablement Zn²⁺, Mn²⁺, Cu²⁺ et Fe²⁺; et un stabilisant de pH, de préférence H₃PO₄ et/ou NaHCO₃.

7. Composition selon l'une quelconque des revendications 3 à 6, où la composition comprend:
(i) le *Trichoderma harzianum* EMPA 720 vivant ou le *Trichoderma koningiopsis* EMPA 723 vivant;
(ii) des granulés de blé d'Inde; et
(iii) un biocharbon, de préférence un biocharbon de bois choisi dans le groupe constitué de: hêtres véritables, érables et frênes, de préférence préparé à des températures entre 450°C et 500°C,
dans laquelle un gramme de (ii) est inoculé avec 10⁴-10⁸ spores de *Trichoderma harzianum* EMPA 720 vivant ou de *Trichoderma koningiopsis* EMPA 723 vivant et dans laquelle le rapport en poids des composants (ii) sur (iii) est de préférence au moins environ 1: 10, plus préférablement environ 5: 10, le plus préférablement de 1:10 à 1:1.

8. Utilisation de *Trichoderma harzianum* EMPA 720 vivant ou de *Trichoderma koningiopsis* EMPA 723 vivant selon la revendication 1 ou 2 ou d'une composition selon l'une quelconque des revendications 3 à 7 pour le traitement d'un sol et/ou d'un matériel végétal contre:
(a) une pourriture de plante et/ou de bois, et/ou
(b) une infestation de champignons résistant au cuivre dans un sol et/ou un matériel végétal.

9. Utilisation selon la revendication 8, dans laquelle le matériel végétal est choisi dans le groupe constitué de: plantes cultivées, de préférence tomates, concombres, aubergines, fraises, plants de cacao, palmiers à huile, vignes, blé, plants de thé et plants de café; bois et produits de bois, de préférence choisis dans le groupe constitué de: arbres, bois d'œuvre, poteaux de bois, palissades de bois et préparation de bois.

10. Utilisation selon l'une quelconque des revendications 8 ou 9, dans laquelle le matériel végétal est un matériel végétal traité au cuivre et/ou au chrome, de préférence un poteau de bois ou une préparation de bois.

11. Méthode pour le traitement d'un sol et/ou d'un matériel végétal contre (a) une pourriture de plante et/ou de bois, et/ou (b) une infestation de champignons résistant au cuivre dans un sol et/ou un matériel végétal, comprenant les étapes de:
(i) fourniture de *Trichoderma harzianum* EMPA 720 vivant ou de *Trichoderma koningiopsis* EMPA 723 vivant selon la revendication 1, de spores selon la revendication 2 et/ou d'une composition selon l'une quelconque des revendications 3 à 7; et
(ii) application du champignon, des spores et/ou de la composition de (i) sur le sol et/ou le matériel végétal.

12. Méthode selon la revendication 11, dans laquelle le champignon, les spores et/ou la composition de (i) sont appliqués sur un sol au voisinage du matériel végétal intéressant, de préférence à une distance de 1 m, plus préférablement d'au maximum 2, 5, 10 ou 15 m, le plus préférablement d'au maximum 20 m.

13. Méthode selon l'une quelconque des revendications 11 ou 12, dans laquelle le matériel végétal est choisi dans le groupe constitué de: plantes cultivées, de préférence tomates, concombres, aubergines, fraises, plants de cacao, palmiers à huile, vignes, blé, plants de thé et plants de café; bois et produits de bois, de préférence choisis dans le groupe constitué de: arbres, bois d'œuvre, poteaux de bois, palissades de bois et préparation de bois.

14. Méthode selon l'une quelconque des revendications 11 à 13, dans laquelle le matériel végétal est un matériel végétal traité au cuivre et/ou au chrome, de préférence un poteau de bois ou une préparation de bois.

15. Méthode selon l'une quelconque des revendications 11 à 14, dans laquelle la composition comprend au moins un composé véhicule physiologiquement acceptable choisi dans le groupe constitué de:
(i) un charbon, de préférence un charbon choisi dans le groupe constitué de: biocharbon, biocharbon activé, charbon d'arbres caducs, de préférence des arbres choisis dans le groupe constitué de: hêtres véritables, frênes, érables, chênes, saules, aubépines, noisetiers et aulnes; plus préférablement un charbon préparé à plus de 200°C, de préférence plus de 300°C, plus préférablement plus de 400°C, le plus préférablement entre 400 et 700°C;
(ii) granulés de blé d'Inde ou granulés de maïs,
(iii) un compost, de préférence un compost végétal, plus préférablement un compost de feuilles,
(iv) un sol pour fleurs,
(v) une poudre de talc,
(vi) vermiculite,
(vii) une poussière de bois ou des copeaux de bois, de préférence une poussière de bois ou des copeaux de bois provenant de bois durs, plus préférablement une poussière de bois ou des copeaux de bois provenant d'arbres caducs, de préférence provenant d'arbres choisis dans le groupe constitué de: *Picea abies*, *Abies alba*, *Fagus sylvatica*, *Fraxinus excelsior* et *Acer pseudoplatanus;* et
(viii) mélanges de l'un quelconque de (i) à (vii).

16. Méthode selon l'une quelconque des revendications 11 à 15 pour la prévention et/ou le traitement d'une infestation de champignons résistant au cuivre dans un sol et/ou un matériel végétal.
